(19) Europäisches Patentamt
European Patent Office
Office européen des brevets

(11) **EP 4 535 220 A1**

(12) **EUROPEAN PATENT APPLICATION**
published in accordance with Art. 153(4) EPC

(43) Date of publication:
**09.04.2025 Bulletin 2025/15**

(21) Application number: **23815521.2**

(22) Date of filing: **16.03.2023**

(51) International Patent Classification (IPC):
**G06F 30/27** (2020.01)          **C04B 38/00** (2006.01)
**G06F 30/10** (2020.01)

(52) Cooperative Patent Classification (CPC):
**C04B 38/00; G06F 30/10; G06F 30/27**

(86) International application number:
**PCT/JP2023/010484**

(87) International publication number:
**WO 2023/233759 (07.12.2023 Gazette 2023/49)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.05.2022   JP 2022089036**

(71) Applicants:
• **NGK Insulators, Ltd.
  Nagoya city, Aichi 467-8530 (JP)**
• **Institute of Science Tokyo
  Tokyo 152-8550 (JP)**

(72) Inventors:
• **SOKAWA, Shingo
  Nagoya-city, Aichi 467-8530 (JP)**

• **HASHIMOTO OKA Yuki
  Nagoya-city, Aichi 467-8530 (JP)**
• **TAKAHASHI, Tomonori
  Nagoya-city, Aichi 467-8530 (JP)**
• **OKAWARA, Shinichi
  Tokyo 152-8550 (JP)**
• **YASUDA, Tomoki
  Tokyo 152-8550 (JP)**
• **MATSUDA, Yosuke
  Tokyo 152-8550 (JP)**
• **YOSHIKAWA, Shiro
  Tokyo 152-8550 (JP)**
• **MATSUMOTO, Hideyuki
  Tokyo 152-8550 (JP)**

(74) Representative: **Mewburn Ellis LLP
Aurora Building
Counterslip
Bristol BS1 6BX (GB)**

(54) **METHOD FOR DESIGNING POROUS BODY AND METHOD FOR MANUFACTURING POROUS BODY**

(57)     A porous body design method using a computer causes the computer to execute, a plurality of times, a characteristic prediction/calculation process of predicting or calculating characteristics of a porous body having a predetermined three-dimensional structure, an evaluation process of evaluating the characteristics of the porous body predicted or calculated by the characteristic prediction/calculation process, and an optimization process of changing connectivity between pores in the three-dimensional structure to search for an optimal connectivity, in which the three-dimensional structure of the porous body is determined on the basis of evaluation results of the characteristics of the porous body by the evaluation process.

**EP 4 535 220 A1**

## FIG. 2

```
                    START

i=0, j=0                                    S10

INITIAL LEARNING MODEL                      S20
GENERATION PROCESS

ACQUIRE THREE-DIMENSIONAL                   S30
STRUCTURE OF TARGET
POROUS BODY

CONVERT THREE-DIMENSIONAL                   S40
STRUCTURE INTO PN MODEL

SET INITIAL VALUES OF                       S50
NETWORK GENE VECTOR

RECONFIGURE THREE-                          S55
DIMENSIONAL STRUCTURE

CALCULATE VALUES OF INPUT                   S60
PARAMETERS

        i=M?        S70      YES →   PERFORM CHARACTERISTIC      S100
                                     CALCULATION BY PHYSICAL
         NO                          SIMULATION

PERFORM CHARACTERISTIC       S80     UPDATE LEARNING MODEL       S110
PREDICTION BY AI CALCULATION

i=i+1                        S90     i=0                         S120

PLOT CHARACTERISTIC                         S130
PREDICTION/CALCULATION
RESULTS

SELECT PARENT POROUS                S135     GENERATE NEXT               S160
BODIES OF NEXT GENERATION                    CANDIDATE NETWORK
                                             GENE VECTOR

        j=N?        S140     NO →    RECONFIGURE THREE-          S170
                                     DIMENSIONAL STRUCTURE
         YES

DETERMINE THREE-DIMENSIONAL  S150    j=j+1                       S180
STRUCTURE OF POROUS BODY

                    END
```

## Description

[Technical Field]

The present invention relates to a porous body design method and a porous body manufacturing method.

[Background Art]

In the related art, porous bodies in which many pores are disposed three-dimensionally in a material have been widely used for various applications, such as purification of exhaust gases emitted from internal combustion engines such as gasoline and diesel engines. In designing such porous bodies, it is necessary to determine a three-dimensional structure of a porous body so that appropriate characteristics can be obtained in accordance with the application.

Porous body design methods of the related art include, for example, a method of creating several prototypes of porous bodies on the basis of a designer's experience and intuition, measuring a three-dimensional structure and characteristics of each of these prototypes, and then determining a final three-dimensional structure of the porous body after ascertaining a relationship between the three-dimensional structure and the characteristics of the porous body. However, with such a design method, it takes a lot of time and laborious effort to create prototypes and measure the characteristics, and thus there is a limitation on a range in which a relationship between a three-dimensional structure and characteristics can be searched for. For this reason, there is a problem in that it is not necessarily possible to determine a three-dimensional structure of a porous body from which desired characteristics can be obtained.

On the other hand, in recent years, a method using simulation software has also become known, for example, as disclosed in PTL 1. With such simulation software, a three-dimensional model of a porous body can be created, and characteristics of the porous body can be obtained from this three-dimensional model by calculation.

[Citation List]

[Patent Literature]

[PTL 1] Japanese Patent No. 6940786

[Summary of Invention]

[Technical Problem]

In the method disclosed in PTL 1, a sample is created from a partition piece cut out from an exhaust gas purification filter, and continuous cross-sectional images, which are obtained by imaging the sample with an X-ray CT scanner, are read by simulation software to form a three-dimensional model, thereby deriving the number of continuous holes of the exhaust gas purification filter, i.e., a porous body. Thus, it is necessary to create the exhaust gas purification filter in advance, and there is still a problem in that a lot of time and laborious effort are required for prototyping.

In view of the above-mentioned problems, an object of the present invention is to provide a useful technology that makes it possible to determine an optimal structure early in the design of a porous body.

[Solution to Problem]

A porous body design method according to the present invention is a design method of porous body using a computer, and causes the computer to execute, a plurality of times, a characteristic prediction/calculation process of predicting or calculating characteristics of a porous body having a predetermined three-dimensional structure, an evaluation process of evaluating the characteristics of the porous body predicted or calculated by the characteristic prediction/calculation process, and an optimization process of changing connectivity between pores in the three-dimensional structure to search for an optimal connectivity, in which the three-dimensional structure of the porous body is determined based on evaluation results of the characteristics of the porous body by the evaluation process.

A porous body manufacturing method according to the present invention is a method of manufacturing the porous body using three-dimensional shape data based on the three-dimensional structure of the porous body determined by the porous body design method.

[Advantageous Effects of Invention]

According to the present invention, it is possible to provide a useful technology that makes it possible to determine an optimal structure early in the design of a porous body.

[Brief Description of Drawings]

[Fig. 1]

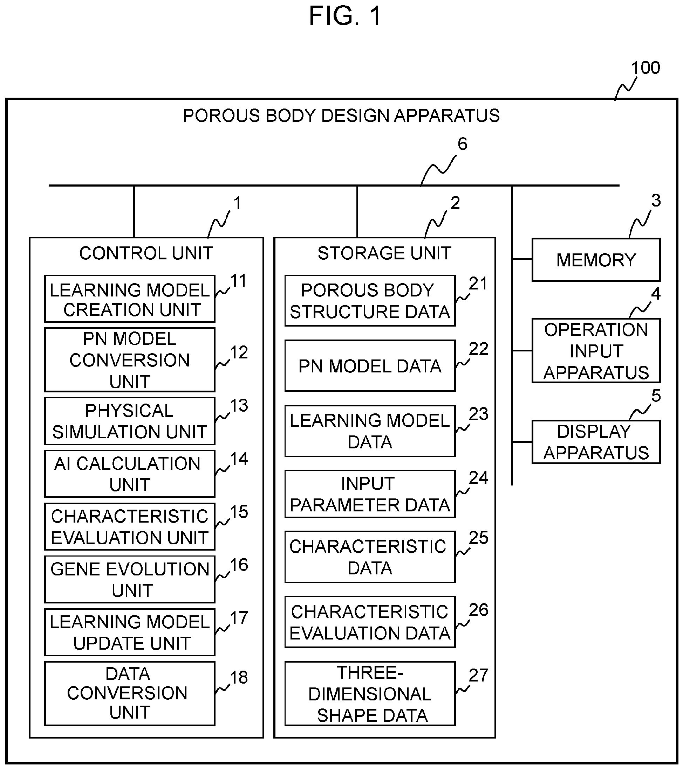

Fig. 1 is a block diagram showing a configuration of a porous body design apparatus according to an embodiment of the present invention.
[Fig. 2]

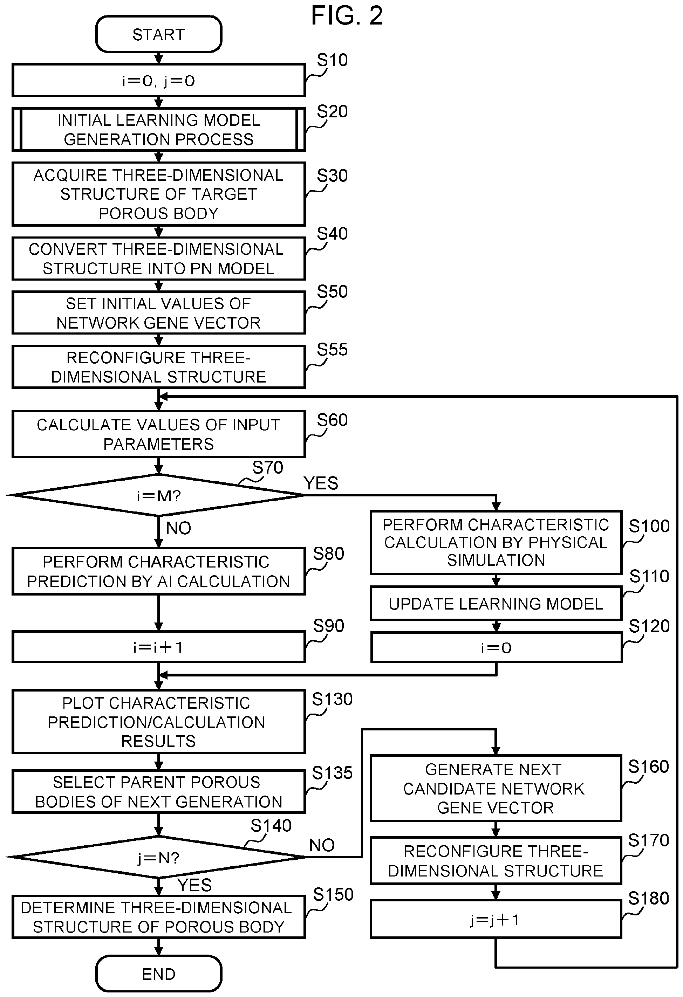

Fig. 2 is a flowchart showing a flow of processing of a porous body design apparatus according to a first embodiment of the present invention.
[Fig. 3]

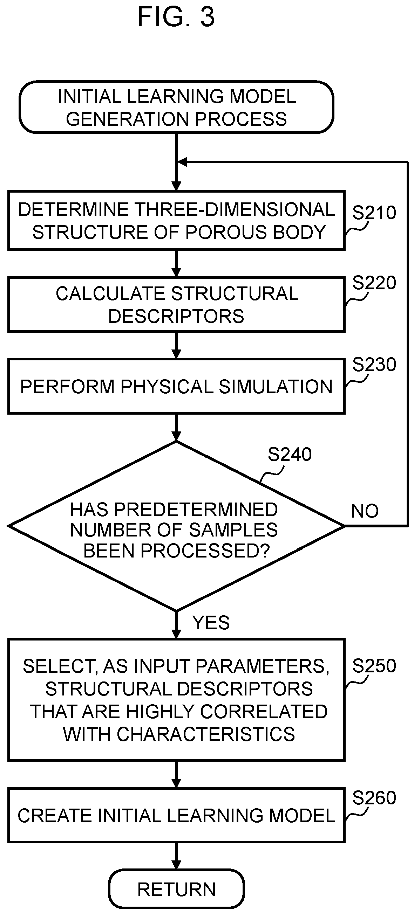

Fig. 3 is a flowchart showing details of an initial learning model generation process according to the first embodiment of the present invention.

[Fig. 4]

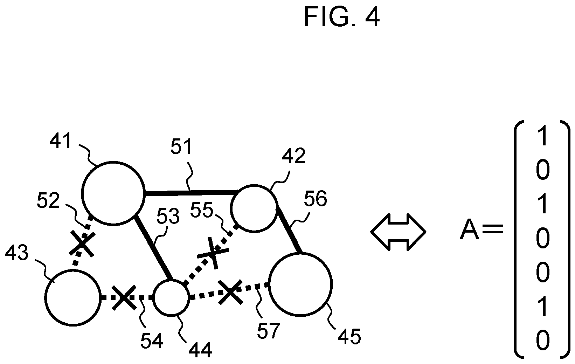

Fig. 4 is a diagram showing an example of a PN model.
[Fig. 5]

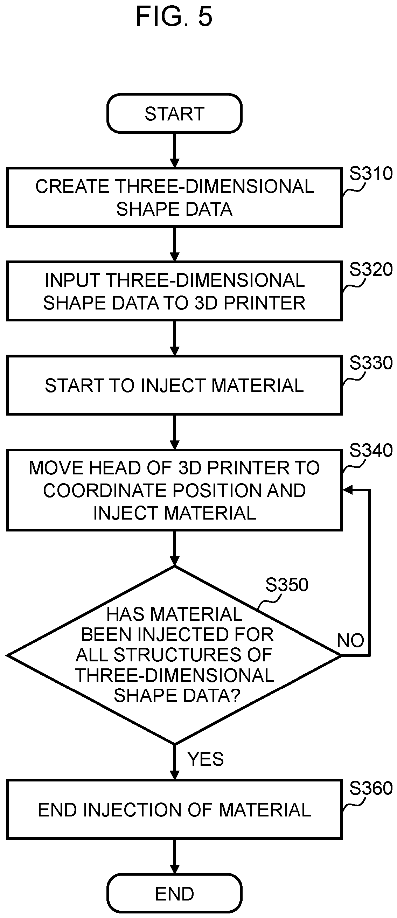

Fig. 5 is a flowchart showing a flow of a porous body manufacturing process using a 3D printer.
[Fig. 6]

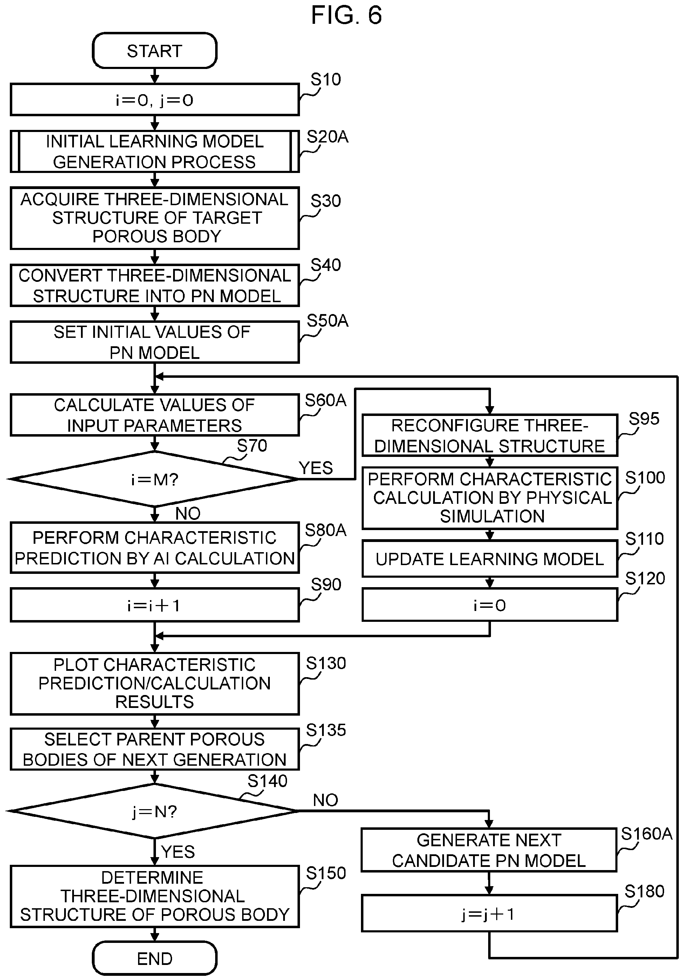

Fig. 6 is a flowchart showing a flow of processing of a porous body design apparatus according to a second embodiment of the present invention.
[Fig. 7]

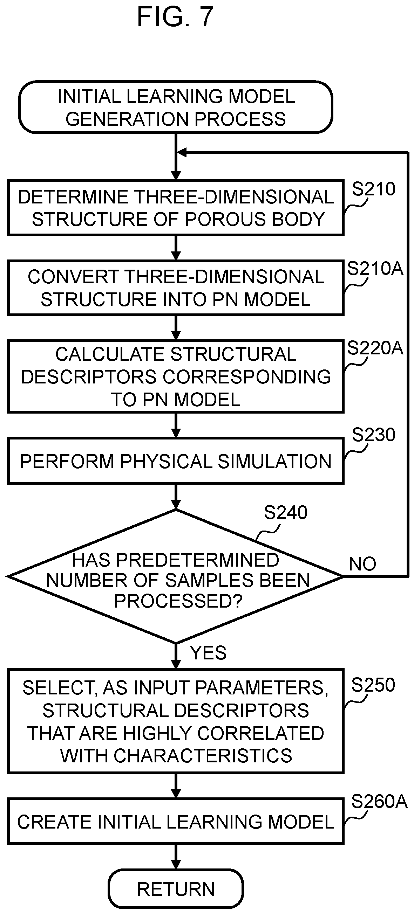

Fig. 7 is a flowchart showing details of an initial learning model generation process according to the second embodiment of the present invention.

[Description of Embodiments]

Hereinafter, embodiments of the present invention will be described with reference to the drawings. In the following embodiments, description will be given of a porous body design apparatus that can determine a three-dimensional structure of a porous body having desired characteristics in a relatively short period of time on the basis of the existing three-dimensional structure of a porous body by being operated by a designer who designs the porous body.

(First embodiment)

Fig. 1 is a block diagram showing a configuration of a porous body design apparatus according to an embodiment of the present invention. A porous body design apparatus 100 shown in Fig. 1 is a computer that includes a control unit 1, a storage unit 2, a memory 3, an operation input apparatus 4, and a display apparatus 5, and is configured by connecting these apparatuses to each other via a bus 6.

The control unit 1 is configured using, for example, a central processing unit (CPU) and performs various processes and calculations for operating the porous body design apparatus 100. The control unit 1 executes programs stored in the storage unit 2 to realize functional blocks of a learning model creation unit 11, a PN model conversion unit 12, a physical simulation unit 13, an AI calculation unit 14, a characteristic evaluation unit 15, a gene evolution unit 16, a learning model update unit 17, and a data conversion unit 18. Details of these functional blocks will be described later. Some or all of the functions of the control unit 1 may be realized using devices other than the CPU, such as a graphics processing unit (GPU), a field programmable gate array (FPGA), and an application specific integrated circuit (ASIC).

The storage unit 2 is configured using a magnetic storage apparatus such as a hard disk drive (HDD) or a large-capacity non-volatile memory apparatus such as a solid state drive (SSD), and stores programs executed by the control unit 1 and various types of information used in the processing of the control unit 1. The information stored in the storage unit 2 includes data including porous body structure data 21, PN model data 22, learning model data 23, input parameter data 24, characteristic data 25, characteristic evaluation data 26, and three-dimensional shape data 27. Details of the data will be described later.

The memory 3 is configured using a high-speed volatile storage apparatus such as a dynamic random access memory (DRAM), and is used as a working area when the control unit 1 executes a program.

The operation input apparatus 4 is an apparatus for detecting a user's operation input and is configured using, for example, a keyboard or a mouse. The display apparatus 5 is an apparatus that displays processing results of the porous body design apparatus 100 on a screen and presents them to the user, and is configured using, for example, a liquid crystal display or an organic EL display. Another computer or smartphone that can communicate with the porous body design apparatus 100 may be used as the operation input apparatus 4 or the display apparatus 5.

The configuration of the porous body design apparatus 100 shown in Fig. 1 may be physically constructed on one computer or may be constructed to be distributed on a plurality of computers. The porous body design apparatus 100 may also be realized by a cloud computer installed on a cloud, a virtual machine operating in a virtual environment, or the like.

Next, the functional blocks of the control unit 1, that is, the learning model creation unit 11, the PN model conversion unit 12, the physical simulation unit 13, the AI calculation unit 14, the characteristic evaluation unit 15, the gene evolution unit 16, the learning model update unit 17, and the data conversion unit 18, will be described. In the porous body design apparatus 100, the control unit 1 operates as these functional blocks, making it possible to determine a three-dimensional structure of a porous body having desired characteristics.

The learning model creation unit 11 creates a learning model capable of predicting characteristics corresponding to the three-dimensional structure of the porous body. The learning model is a prediction model that learns a relationship between the three-dimensional structure of the porous body and the characteristics, and is also referred to as a surrogate model. For example, by using a support vector machine (SVM), it is possible to generate a support vector regression that makes it possible to predict the characteristics of the porous body from feature data of the three-dimensional structure of the porous body as a learning model. A method of constructing a learning model using an SVM is known, and is described, for example, by Tomoki Yasuda, Shinichi Ookawara, Shiro Yoshikawa, and Hideyuki Matsumoto in "Machine learning and data-driven characterization framework for porous materials: Permeability prediction and channeling defect detection", Chemical Engineering Journal 420 (2021) 130069 DOI: 10.1016/j.cej.2021.130069. Information on the learning model created by the learning model creation unit 11 is stored in the storage unit 2 as learning model data 23. A learning model acquired from the outside may be stored in the storage unit 2 as the learning model data 23.

When a learning model is created, the learning model creation unit 11 acquires a predetermined number of samples having a three-dimensional structure of a porous body in advance. Then, various structural features of the samples are expressed by the values of a plurality of types of structural descriptors determined in advance. Structural descriptors that represent a three-dimensional structure of a porous body include, for example, a porosity, a solid fraction, a specific surface area, a pore diameter, a particle diameter, structural uniformity of pores or solid, a pore cord length, a solid cord length, a pore-throat ratio, a pore-throat coordination number, and the like. More specifically, the structural uniformity of the pores or solid means the variance of values related to the pores or solid calculated for each area by dividing the porous body into a plurality of areas. Examples of the values related to the pores or solid calculated for each area include the above-mentioned porosity, solid fraction, specific surface area, pore diameter, particle diameter, and the like. The pore cord length is the continuous length of pores in a predetermined direction in the porous body, and the solid cord length is the continuous length of the solid in a predetermined direction in the porous body. The pore-throat ratio is a ratio between the diameter of a pore and a throat, which will be described later, and the pore-throat coordination number is an average value of the number of throats between adjacent pores for each pore. The learning model creation unit 11 selects, from among these structural descriptors, a plurality of structural descriptors that contribute highly to the characteristics of the porous body as input parameters for the learning model. Then, a learning model that predicts characteristics from the input parameters is created using the value of each input parameter of each sample and the characteristics of the porous body obtained for each sample by the following physical simulation performed by the physical simulation unit 13.

The PN model conversion unit 12 converts the three-dimensional structure of the porous body into a pore-throat network (PN) model. The PN model is a model in which a three-dimensional structure of a porous body is modeled by combining parts equivalent to pores, referred to as pores, with connecting parts between pores, referred to as throats. That is, in the PN model, the three-dimensional structure of the porous body is expressed using pores which represent the position and size of each pore formed inside the porous body, throats which represent a network between pores related to connectivity between the pores, and the thickness (diameter) of each throat. Here, as will be described in detail later, in the porous body design apparatus 100 of this embodiment, information on each throat between pores in the PN model is treated as vector information referred to as a network gene vector. Thereby, it is possible to easily change the connectivity between pores in the three-dimensional structure of the porous body to a value represented by any vector information. Information on the PN model created by converting the three-dimensional structure of the porous body by the PN model conversion unit 12 is stored in the storage unit 2 as the PN model data 22.

In addition, the PN model conversion unit 12 can also reconfigure a three-dimensional structure of a porous body corresponding to the PN model by reading out the PN model data 22 stored in the storage unit 2 and inversely converting the PN model represented by the PN model data 22 into the three-dimensional structure of the porous body. The PN model conversion unit 12 can perform, for example, inverse conversion from the PN model to the three-dimensional structure of the porous body by using a well-known algorithm such as pix2pix. Information on the three-dimensional structure of the porous body obtained by inversely converting the PN model by the PN model conversion unit 12 is stored in the storage unit 2 as the porous body structure data 21.

The physical simulation unit 13 performs physical calculations based on the three-dimensional structure of the porous body represented by the porous body structure data 21, and performs a physical simulation when a predetermined material is passed through the porous body. Thereby, for example, when a fluid such as exhaust gas from an internal combustion engine is passed through the porous body, values such as the permeability of the fluid and filtration efficiency (capture efficiency) of particulate matter (PM) contained in the fluid can be calculated as the characteristics of the porous body. In addition, the characteristics of the porous body to be calculated may include mechanical strength characteristics, electrochemical characteristics, thermal conductivity characteristics, heat exchange characteristics, electrical conductivity characteristics, gas adsorption characteristics, gas purification performance, catalyst coating properties, and removal efficiency of substances captured in the porous body. Results of the calculation of the characteristics of the porous body by the physical simulation unit 13 are stored in the storage unit 2 as the characteristic data 25. The physical simulation unit 13 can be realized using, for example, Ansys Fluent, which is thermal fluid analysis software manufactured by ANSYS, Inc. In addition, the permeability and filtration efficiency can also be calculated respectively using a FlowDict module and a FilterDict module included in GeoDict which is microstructure simulation software developed by Math2Market GmbH.

The AI calculation unit 14 performs calculation related to characteristic prediction of the porous body by artificial intelligence (AI) by using the learning model created by the learning model creation unit 11. The AI calculation unit 14 inputs the values of the above-mentioned input parameters obtained for the three-dimensional structure of the porous body into a learning model, thereby performing AI calculations using a well-known AI method such as support vector regression, and can predict the characteristics of the porous body. The values of the input parameters used in the AI calculation unit 14 and the obtained characteristic prediction results for the porous body are stored in the storage unit 2 as the input parameter data 24 and the characteristic data 25, respectively.

The characteristic evaluation unit 15 performs evaluation processing for evaluating the characteristics of the porous body calculated or predicted by the physical simulation unit 13 or the AI calculation unit 14. The characteristic evaluation unit 15 performs evaluation processing for the characteristics of the porous body, for example, by plotting each calculated or predicted characteristic value on a graph. Other methods may be used as long as the characteristics of the porous body can be appropriately evaluated. For example, an evaluation score for the characteristics of the porous body may be calculated from each characteristic value, or it may be determined whether each characteristic value satisfies a predetermined standard value and an OK/NG determination may be made for the characteristics of the porous body on the basis of the determination result. The characteristic evaluation results for the porous body which are obtained by the characteristic evaluation unit 15 are stored in the storage unit 2 as the characteristic evaluation data 26.

The gene evolution unit 16 performs processing for changing connectivity between pores in the PN model. The gene evolution unit 16 performs a gene evolution process using a well-known calculation method such as a genetic algorithm, thereby changing the above-mentioned network gene vector in the PN model data 22 to improve the characteristics of the porous body having a three-dimensional structure of connectivity corresponding to the network gene vector. By the gene evolution unit 16 repeating such a gene evolution process, it is possible to successively update the content of the PN model represented by the PN model data 22 and to search for the optimal connectivity.

The learning model update unit 17 performs an update process for the learning model on the basis of the results of the physical simulation performed by the physical simulation unit 13. As will be described below, the physical simulation unit 13 performs a physical simulation based on the three-dimensional structure of the porous body each time the AI calculation unit 14 performs an AI calculation a predetermined number of times. When the physical simulation unit 13 performs a physical simulation in this manner, the learning model update unit 17 performs an update process for the learning model by using the results and updates the content of the learning model data 23. Thereby, the results of the physical simulation are reflected in the learning model, and the learning model data 23 can be updated such that the values of the characteristics obtained by the AI calculation from the input parameter values according to the three-dimensional structure of the porous body become more accurate.

The data conversion unit 18 converts the porous body structure data 21 based on the three-dimensional structure of the porous body having the finally determined connectivity between pores into three-dimensional shape data 27. The three-dimensional shape data 27 generated by conversion from the porous body structure data 21 by the data conversion unit 18 is stored in the storage unit 2, and is read out from the storage unit 2 as necessary and provided to the outside of the porous body design apparatus 100. For example, the three-dimensional shape data 27 is input to a 3D printer, thereby operating the 3D printer in accordance with the three-dimensional shape data 27 to manufacture a porous body that reproduces the three-dimensional structure of the porous body represented by the porous body structure data 21. Thereby, it is possible to create a prototype of the porous body designed by the porous body design apparatus 100. The details of a manufacturing method for a porous body by the 3D printer using the three-dimensional shape data 27 will be described later.

Next, details of processing performed by the control unit 1 will be described with reference to Fig. 2. Fig. 2 is a flowchart showing a flow of processing of the porous body design apparatus according to the first embodiment of the present invention. The porous body design apparatus 100 of this embodiment searches for a three-dimensional structure of a porous body having desired characteristics by repeatedly executing the processing shown in the flowchart of Fig. 2 by the control unit 1 in response to a user's operation input. Thereby, the design of a porous body which is performed by the user is supported.

When the user instructs the porous body design apparatus 100 to start to design a porous body via the operation input apparatus 4, the control unit 1 sets an initial value 0 to each of variables i and j in step S10. The variable i is a variable for counting the number of times the AI calculation unit 14 has performed an AI calculation between the time when the physical simulation unit 13 performs a physical simulation and the time when it next performs a physical simulation. The variable j is a variable for counting the number of times the gene evolution unit 16 has performed a gene evolution process, that is, the number of generations of the network gene vectors that have been generated so far by the gene evolution process. While the processing shown in the flowchart of Fig. 2 is being performed, the values of the variables i and j are stored in the memory 3.

In step S20, the control unit 1 performs an initial learning model generation process for setting the initial state of the learning model data 23. Here, a large number of samples of the three-dimensional structure of the porous body are acquired using the learning model creation unit 11 and the physical simulation unit 13, and a relationship between the values of the input parameters selected from among the structural descriptors of the samples and the characteristics is obtained. Then, the initial state of the learning model data 23 can be set by creating a learning model on the basis of the obtained relationship. Details of the initial learning model generation process in this embodiment will be described later with reference to Fig. 3.

In step S30, the control unit 1 acquires a three-dimensional structure of a target porous body. The three-dimensional structure of the target porous body is a three-dimensional structure of a porous body that is focused on as a search starting point when the porous body design apparatus 100 searches for a three-dimensional structure of a porous body having desired characteristics. For example, an existing porous body can be taken as a target porous body, and a three-dimensional structure obtained by imaging the target porous body with a computed tomography (CT) apparatus, a three-dimensional structure virtually generated on the porous body design apparatus 100 using the above-mentioned GeoDict, or the like can be acquired as the three-dimensional structure of the target porous body. When the three-dimensional structure of the target porous body has been acquired, the information is stored in the storage unit 2 as porous body structure data 21, and the processing proceeds to step S40.

In step S40, the control unit 1 causes the PN model conversion unit 12 to convert the three-dimensional structure of the target porous body acquired in step S30 into a PN model. Here, the three-dimensional structure of the target porous body can be converted into a PN model, for example, as described below.

Fig. 4 is a diagram showing an example of a PN model. In Fig. 4, circles indicated by reference numerals 41 to 45 indicate pores included in a target porous body. In addition, line segments indicated by reference numerals 51 to 57 indicate throats or unconnected throats that are present between the pores 41 to 45. The line segments 51, 53, and 56 indicated by solid lines shown in Fig. 4 represent the throats that actually connect the pores, and the line segments 52, 54, 55, and 57 indicated by dashed lines represent the unconnected throats that do not actually connect the pores.

When the PN model conversion unit 12 detects the positions and sizes of the pores 41 to 45 from the three-dimensional structure of the target porous body, the PN model conversion unit 12 extracts combinations of the pores 41 to 45 whose mutual distances are within a predetermined range, and provisionally sets throat candidates 51 to 57 that connect the pores for each combination. Then, among these throat candidates 51 to 57, those that actually connect the pores are set as throats, and those that do not actually connect the pores are set as unconnected throats. As a result, the throats 51, 53, 56 and the unconnected throats 52, 54, 55, 57 are set, and the three-dimensional structure of the target porous body is converted into the PN model shown in Fig. 4.

After the three-dimensional structure of the target porous body is converted into the PN model as described above, the control unit 1 generates an initial value of a network gene vector to which a genetic algorithm is applied in the following step S50 on the basis of the network gene vector corresponding to the PN model. As described above, the network gene vector is a vector that expresses information on each throat between pores in the PN model by a vector, and each element of the vector represents connection information of each throat of the PN model. Here, for the network gene vector representing the PN model of the target porous body created in step S40, a combination of element values consisting of the same number of vector elements and having the same as or different from the element values of the network gene vector is generated as the initial value of the network gene vector. For example, for the PN model in Fig. 4 which corresponds to the three-dimensional structure of the target porous body, a network gene vector A of a target porous body is created by setting the values of vector elements corresponding to the throats 51, 53, and 56 to "1" and setting the values of vector elements corresponding to the unconnected throats 52, 54, 55, and 57 to "0". Then, the initial value of the network gene vector can be generated by using the element values of the network gene vector A as they are or by changing them arbitrarily.

In step S50, the values of the elements of the network gene vector A are randomly changed by a preset population number (the number of generation individuals) P, and are set as initial values of the network gene vector. For example, when P = 100, 100 combinations of vector element values which consist of the same number of vector element values as the network gene vector A of the target porous body are set as the initial values of the network gene vector. Each porous body represented by this initial value is used as a parent generation for a first generation porous body in a gene evolution process in step S160 to be described below. For this reason, in the following description, the porous body represented by the initial value of the network gene vector is referred to as a porous body of a 0-th generation.

The control unit 1 associates the information on the position and size of each pore in the PN model created in step S40 with the information on the initial values of the network gene vector created in step S50, and stores them in the storage unit 2 as PN model data 22. Thereby, each PN model data 22 representing a three-dimensional structure of a porous body of a 0-th generation and having a different throat structure is stored in the storage unit 2 by the population number P. Thereafter, the processing proceeds to step S55.

In step S55, the control unit 1 causes the PN model conversion unit 12 to reconfigure a three-dimensional structure of each porous body of a 0-th generation from the initial values of the network gene vector created in step S50. Here, P pieces of PN model data 22 recorded in step S50 are read from the storage unit 2, and the positions and sizes of the pores in the PN model represented by each PN model data 22 and combinations of throats connecting the pores are reproduced on the porous body design apparatus 100. This processing can be realized using a well-known algorithm such as pix2pix. Thereby, each PN model represented by the initial values of the network gene vector is inversely converted into a three-dimensional structure of each porous body of a 0-th generation, and a three-dimensional structure of each porous body of a 0-th generation having a different connectivity between pores from the three-dimensional structure of the target porous body can be virtually generated on the porous body design apparatus 100. When the information on the three-dimensional structure of each porous body of a 0-th generation reconfigured in this manner is recorded in the porous body structure data 21, the processing proceeds to step S60.

In step S60, the control unit 1 calculates the values of input parameters corresponding to a three-dimensional structure of each porous body of a j-th generation which is acquired in step S55 (when j = 0) or in step S170 (when j ≥ 1) to be described later. Here, as described above, for example, among structural descriptors such as a porosity, a solid fraction, a specific surface area, a pore diameter, a particle diameter, structural uniformity of pores or solid, a pore cord length, a solid cord length, a pore-throat ratio, and a pore-throat coordination number, each structural descriptor that is selected in advance by the learning model creation unit 11 as a structural descriptor highly contributing to the characteristics of the porous body is used as an input parameter of the learning model, thereby calculating combinations of input parameter values for the three-dimensional structure of the porous body of the j-th generation which is acquired in step S55 or step S170. When the input parameter values calculated for the three-dimensional structure of the porous body of the j-th generation are stored in the storage unit 2 as input parameter data 24, the processing proceeds to step S70.

In step S70, the control unit 1 determines whether the current value of the variable i is a predetermined value M. When i < M, the processing proceeds to step S80. When i = M, the processing proceeds to step S100. The value of M represents the number of times of setting regarding whether to update the learning model by performing a physical simulation for every several AI calculations, and can be set to any number of 1 or more, for example, M = 20.

In step S80, the control unit 1 causes the AI calculation unit 14 to perform characteristic prediction by an AI calculation using the learning model on the basis of the input parameter values calculated in step S60. Here, AI calculation related to characteristic prediction for a porous body is performed by reading out the learning model data 23 to acquire a learning model and inputting the input parameter values obtained from the three-dimensional structure of the porous body of the j-th generation into the learning model. Thereby, the characteristic prediction is performed for the porous body of the j-th generation by obtaining predicted characteristic values corresponding to combinations of the input parameters.

When the predicted characteristic values obtained for the porous body of the j-th generation are stored in the storage unit 2 as characteristic data 25 in this manner, the processing proceeds to step S90.

In step S90, the control unit 1 adds 1 to the value of the variable i, and causes the processing to proceed to step S130.

In step S100, the control unit 1 causes the physical simulation unit 13 to perform a physical simulation based on the three-dimensional structure of the porous body of the j-th generation acquired in step S170 to perform a characteristic calculation for the porous body of the j-th generation and calculate its characteristic value. When characteristic value calculation results obtained for the porous body of the j-th generation in this manner are stored in the storage unit 2 as characteristic data 25, the processing proceeds to step S110.

In step S110, the control unit 1 causes the learning model update unit 17 to update the learning model represented by the learning model data 23 on the basis of the results of the physical simulation performed in step S100. Here, the input parameter data 24 stored in step S60 and the characteristic data 25 stored in step S100 for the porous body subjected to the physical simulation performed up to that point in time are read from the storage unit 2, and the contents (weight values) of the learning model are updated on the basis of a relationship between the input parameter values and characteristic values of the porous body of the j-th generation in this data. When the updated contents of the learning model are recorded in the learning model data 23, the processing proceeds to step S120.

In step S120, the control unit 1 resets the value of the variable i to 0 and causes the processing to proceed to step S130.

In step S130, the control unit 1 causes the characteristic evaluation unit 15 to plot the characteristic values predicted or calculated in step S80 or S100 on a graph and evaluate the characteristic prediction/calculation results for the porous body of the j-th generation. Here, for example, a two-dimensional graph is prepared in which one axis represents a permeability of a fluid, which is one of the characteristics of the porous body, and the other axis represents a filtration efficiency, which is another characteristic of the porous body, and the predicted or calculated characteristic values of the porous body of the j-th generation are plotted on the two-dimensional graph. Thereby, it is possible to evaluate the characteristic prediction/calculation results for the porous body of the j-th generation on the basis of the positions of points plotted on the two-dimensional graph. As long as it is possible to evaluate the characteristic prediction/calculation results, it is not necessary to actually plot the points on the graph, and it is only required to store data indicating the positions of the points on the graph. Alternatively, as described above, it is also possible to evaluate the characteristic prediction/calculation results using a method other than plotting on the graph.

In step S135, the control unit 1 selects parent porous bodies of the next generation on the basis of the evaluation results of the characteristic prediction/calculation results obtained in step 130. Here, for example, the characteristic values predicted or calculated in the previous and current steps S80 or S100 are relatively evaluated from the positions of the points plotted on the graph in the previous and current step S130. Then, from these, a predetermined percentage of porous bodies that correspond to preferable characteristic values, for example, P porous bodies (P is a population number) having characteristic values corresponding to the top 50%, are selected as parent porous bodies of the next generation, that is, parent porous bodies of a (j + 1)-th generation. However, when the process of step S135 is performed for the first time, that is, when j = 0, there are no previous plot points. For this reason, in this case, all of the porous bodies of the 0-th generation represented by the initial values of the network gene vector generated in step S50 are only required to be selected as parent porous bodies of a 1st generation.

In step S140, the control unit 1 determines whether the current value of the variable j is a predetermined value N. When j = N, the processing proceeds to step S150. When j < N, the processing proceeds to step S160. The value of N represents the number of times the gene evolution unit 16 executes a gene evolution process, and can be set to any number of 1 or more, for example, N = 100.

In step S150, the control unit 1 determines a final three-dimensional structure of a target porous body on the basis of the evaluation results for the characteristics of the porous body of each generation which have been obtained so far. Here, for example, the characteristic values predicted or calculated in the current step S80 or S100 are relatively evaluated from the positions of the points plotted on the graph in the immediately preceding step S130, and the most highly evaluated characteristic value is specified. For example, a point whose permeability and filtration efficiency are within a predetermined range and which is located at a position farthest from the origin of the graph in the upper right direction can be extracted, and the characteristic value represented by the point can be specified as the most highly evaluated characteristic value.

When the most highly evaluated characteristic value can be specified in the process of step S150, the control unit 1 determines a three-dimensional structure corresponding to the characteristic value as the final three-dimensional structure of the target porous body, and ends the processing shown in the flowchart of Fig. 2.

In step S160, the control unit 1 causes the gene evolution unit 16 to generate a next candidate network gene vector. Here, the next candidate network gene vector can be generated by performing a gene evolution processing on the basis of the network gene vectors of the porous bodies selected as the parent porous bodies of the next generation in the immediately preceding step S135, and creating network gene vectors of child porous bodies for the parent porous bodies. Specifically, by using a well-known calculation method such as a genetic algorithm, the network gene vectors of the porous bodies of the j-th generation selected in step S135 are multiplied together to generate a network gene vector of a new porous body corresponding to their child as a network gene vector of porous bodies of a (j + 1)-th generation. By repeatedly performing such an operation for the combinations of the plurality of parent porous bodies, any number of network gene vectors of child porous bodies of the next generation are generated. For example, P pairs of parent porous bodies of the j-th generation (P is a population number) are set, and P network gene vectors of child porous bodies of the (j + 1)-th generation are created from these pairs as the next candidate network gene vectors. As a result, it is possible to obtain, by the population number P, three-dimensional structures of porous bodies of the (j + 1)-th generation in which the positions and sizes of the pores remain the same, but only connectivity between the pores is different with respect to the three-dimensional structures of the porous bodies of the j-th generation. When the values of elements of network gene vectors corresponding to the porous bodies of the (j + 1)-th generation which are newly obtained by this gene evolution process are recorded in the PN model data 22, the processing proceeds to step S170.

In step S170, the control unit 1 causes the PN model conversion unit 12 to reconfigure the three-dimensional structures of the porous bodies of the (j + 1)-th generation from the next candidate network gene vectors generated by the gene evolution process in step S160. Here, the P pieces of PN model data 22 recorded in the immediately preceding step S160 are read out from the storage unit 2, and the positions and sizes of the pores in the PN model represented by each pieces of PN model data 22 and combinations of throats connecting the pores are reproduced on the porous body design apparatus 100. This processing can be realized by using a well-known algorithm such as pix2pix, similar to step S55. Thereby, each PN model represented by the network gene vector after the gene evolution process is inversely converted into the three-dimensional structure of the porous body of the (j + 1)-th generation, and a three-dimensional structure of each porous body of the (j + 1)-th generation, which has a different connectivity between pores from the three-dimensional structure of the target porous body, can be virtually generated on the porous body design apparatus 100. When information on the three-dimensional structure of each porous body of the (j + 1)-th generation reconfigured in this manner is recorded in the porous body structure data 21, the processing proceeds to step S180.

In step S180, the control unit 1 adds 1 to the value of the variable j and causes the processing to return to step S60.

After the processing returns to step S60 from step S180, the control unit 1 repeats the process of step S60 and the subsequent processes again. Thereby, a new three-dimensional structure of a porous body with connectivity between pores, which is different from that of the target porous body, is generated on the basis of the network gene vector after the gene evolution process, and characteristic values are predicted or calculated by an AI calculation or a physical simulation. Then, by adding a plot of the obtained characteristic prediction/calculation results on the graph, new evaluation results for characteristic values of porous bodies of the next generation are acquired in addition to the evaluation results for the characteristic values of the porous bodies of the previous generation which have been obtained up to the previous process, and parent porous bodies of the next generation to be used in the gene evolution process are selected from the obtained evaluation results. This series of processes realizes an optimization process for changing connectivity between pores in a three-dimensional structure of a porous body to search for an optimal connectivity between pores.

Fig. 3 is a flowchart showing details of the initial learning model generation process according to the first embodiment of the present invention, which is executed in step S20 in Fig. 2.

In step S210, the control unit 1 causes the learning model creation unit 11 to determine a three-dimensional structure of a porous body to be used to generate an initial learning model. Here, for example, a three-dimensional structure of a porous body which is set in advance as a sample is determined as the three-dimensional structure for generating the initial learning model.

In step S220, the control unit 1 causes the learning model creation unit 11 to calculate the values of structural descriptors corresponding to the three-dimensional structure determined in step S210. Here, for example, the values of structural descriptors for the three-dimensional structure of the porous body determined in step S210 are calculated for various predetermined structural descriptors, such as a porosity, a solid fraction, a specific surface area, a pore diameter, a particle diameter, structural uniformity of pores or solid, a pore cord length, a solid cord length, a pore-throat ratio, and a pore-throat coordination number.

In step S230, the control unit 1 causes the physical simulation unit 13 to perform a physical simulation based on the three-dimensional structure determined in step S210. Here, through the same process as step S100 in Fig. 2, characteristics of a porous body having a three-dimensional structure for generating an initial learning model are calculated by a physical simulation, and characteristic values obtained as a simulation result are acquired.

In step S240, the control unit 1 determines whether the processes of steps S210 to S230 have been performed for a predetermined number of samples. When the processes of steps S210 to S230 have been performed for porous bodies corresponding to the predetermined number of samples, and characteristic values for the samples have been calculated, the processing proceeds to step S250. On the other hand, when the number of three-dimensional structures whose characteristic values have been calculated by the processes of steps S210 to S230 is less than the predetermined number of samples and the characteristic values acquired so far do not reach the number of samples, the processing returns to step S210 and perform the process of step S210 and the subsequent processes again.

In step S250, the control unit 1 causes the learning model creation unit 11 to select, from among the structural descriptors of the samples calculated in step S220, structural descriptors that are highly correlated with the characteristic values of the porous body obtained by the physical simulation in step S230 as input parameters of the learning model. Here, for example, a correlation between the structural descriptor values calculated for each sample and the characteristic values is obtained for each structural descriptor, and a predetermined number of structural descriptors are selected in descending order of obtained correlation values, as input parameters of the learning model. Thereby, structural descriptors that contribute highly to the characteristics of a porous body can be selected as input parameters of the learning model.

In step S260, the control unit 1 causes the learning model creation unit 11 to create an initial learning model on the basis of the input parameters selected in step S250 and the characteristic values of the porous body obtained in step S230. Here, the initial learning model can be created by performing a model learning process using, for example, a well-known method on the basis of a relationship between the input parameters and characteristic values of each sample.

When the initial learning model is created in step S260, the control unit 1 records the contents of the initial learning model in the learning model data 23 and ends the initial learning model generation process shown in the flowchart of Fig. 3. Thereafter, the processing proceeds to step S30 in Fig. 2.

In the flowchart of Fig. 2, the process of step S130 in which the characteristic values predicted or calculated by an AI calculation or a physical simulation are evaluated by a method such as plotting them on a graph, the gene evolution process of step S160, and the process of step S170 in which the three-dimensional structure of the porous body is reconfigured from the PN model of the network gene vector after the gene evolution process are repeatedly performed to finally obtain a large number of plot points that represent the respective characteristic values on a graph for porous bodies having various three-dimensional structures with different connectivity between pores. In step S150, a point that shows the best characteristics among these plot points is selected as the most highly evaluated characteristic value. The three-dimensional structure corresponding to this characteristic value is then specified to determine the final three-dimensional structure of the target porous body. Even when the points are not actually plotted on the graph as described above, but data showing the positions of the points on the graph is stored, it is possible to select a point that shows the best characteristics using a similar method and specify a three-dimensional structure corresponding to the point to determine the final three-dimensional structure of the target porous body.

Here, a connectivity index between pores in the original three-dimensional structure of the target porous body and its characteristic values are compared with a connectivity index between pores in the three-dimensional structure of the target porous body after optimization which are finally determined and its characteristic values when the processing shown in the flowchart in Fig. 2 is repeated a predetermined number of times, for example 100 times. An example of the comparison results is shown below. In the following description, the connectivity index between pores in the original three-dimensional structure of the target porous body and its characteristic values (permeability, filtration efficiency) are each set to 100, and the three-dimensional structure of the target porous body after optimization and the characteristic values are normalized and shown. Here, the larger the connectivity index value, the greater the number of throats connecting pores, which indicates that the proportion of connected throats among throat candidates in the PN model is large and indicates that the higher the values of permeability and filtration efficiency, the better characteristics.

Original target porous body: connectivity index = 100, permeability = 100, filtration efficiency = 100 Target porous body after optimization: connectivity index = 108, permeability = 122, filtration efficiency = 105

From the above comparison results, it can be understood that the connectivity index value of the target porous body after optimization is higher than that of the original target porous body, which leads to good characteristics. Thus, it can be understood that it is possible to adjust the connectivity between pores to an optimal value to determine the three-dimensional structure of a porous body having desired characteristics by adopting a porous body design method using the porous body design apparatus 100 of this embodiment.

Next, details of a method of manufacturing a porous body by a 3D printer using the three-dimensional shape data 27 will be described with reference to Fig. 5. Fig. 5 is a flowchart showing a flow of a manufacturing process for a porous body using a 3D printer.

When a user instructs the porous body design apparatus 100 to start to manufacture a porous body via the operation input apparatus 4, the control unit 1 causes the data conversion unit 18 to create three-dimensional shape data 27 in step S310. Here, the porous body structure data 21 is read out from the storage unit 2 and is subjected to a predetermined data conversion process, thereby converting the porous body structure data 21 into the three-dimensional shape data 27. The porous body structure data 21 may be used as the three-dimensional shape data 27 as it is. In this case, the control unit 1 may not include the data conversion unit 18.

In step S320, the three-dimensional shape data 27 created in step S310 is input to a 3D printer (not shown). Here, for example, the porous body design apparatus 100 and the 3D printer are connected in a wireless or wired manner, and the three-dimensional shape data 27 is transmitted from the porous body design apparatus 100 to the 3D printer, thereby inputting the three-dimensional shape data 27 to the 3D printer. Alternatively, the three-dimensional shape data 27 may be input to the 3D printer by moving the three-dimensional shape data 27 from the porous body design apparatus 100 to the 3D printer via a storage medium such as a USB memory. In addition to this, the three-dimensional shape data 27 can be input to the 3D printer by any method.

In step S330, the 3D printer starts to inject a material for forming a porous body on the basis of the three-dimensional shape data 27 input in step S320. Then, in step S340, the head of the 3D printer is moved to the coordinate position represented by the three-dimensional shape data 27 input in step S320, and then the material is injected.

In step S350, it is determined whether a material has been injected from the 3D printer for all three-dimensional structures of the porous body represented by the three-dimensional shape data 27 input in step S320. When there are any three-dimensional structure parts for which the material has not been injected, the processing returns to step S340 to continue injecting the material, and when the material has been injected for all three-dimensional structures, the processing proceeds to step S360.

In step S360, the injection of the material in the 3D printer is ended. Thereby, the manufacture of the porous body using the 3D printer is completed, and a porous body that reproduces the three-dimensional structure designed by the porous body design apparatus 100 can be created. After the process of step S360 is executed, the processing shown in the flowchart of Fig. 5 is ended.

Although an example of a method for manufacturing a porous body by a 3D printer using the three-dimensional shape data 27 has been described above, a porous body that reproduces the three-dimensional structure designed by the porous body design apparatus 100 may be manufactured by other methods. Alternatively, a porous body may be manufactured using something other than a 3D printer. When a porous body that reproduces the three-dimensional structure designed by the porous body design apparatus 100 can be appropriately manufactured using the three-dimensional shape data 27, any method can be selected from among various well-known methods and used to manufacture the porous body.

According to the first embodiment of the present invention described above, the following operational effects are achieved.

(1) A method of designing a porous body using the porous body design apparatus 100 causes the porous body design apparatus 100 to execute, a plurality of times, a characteristic prediction/calculation process (steps S80, S100) of predicting or calculating the characteristics of a porous body having a predetermined three-dimensional structure, an evaluation process (step S130) of evaluating the characteristics of the porous body predicted or calculated by the characteristic prediction/calculation process, and an optimization process (steps S135, S160) of changing connectivity between pores in the three-dimensional structure to search for an optimal connectivity. Then, the three-dimensional structure of the porous body is determined on the basis of the evaluation results of the characteristics of the porous body through the evaluation process (step S150). In this manner, it is possible to provide a useful technology that makes it possible to determine an optimal structure early in the design of a porous body.
(2) In a porous body design method, the porous body design apparatus 100 executes a PN modeling process (step S40) for generating a PN model that expresses a three-dimensional structure using a plurality of pores and a plurality of throats that connect the pores. In the process of step S160, connectivity is changed by adding or deleting throats in the PN model generated in the PN modeling process of step S40. In this manner, it is possible to easily change the connectivity between pores in a three-dimensional structure.
(3) In the characteristic prediction/calculation process, the characteristics of the porous body are predicted on the basis of the PN model generated in the PN modeling process of step S40. Specifically, the porous body design apparatus 100 executes a structure generation process (step S170) in which a three-dimensional structure of the porous body based on the PN model to which throats have been added or deleted in the process of step S160 is virtually generated on the porous body design apparatus 100. In the characteristic prediction process of step S80, the characteristics of the porous body having the three-dimensional structure generated by the structure generation process are predicted. In this manner, it is possible to reliably predict the characteristics of the porous body in which connectivity between pores has been changed by the optimization process.
(4) Further, in the characteristic prediction/calculation process, at least one of a physical simulation (step S100) and an AI calculation (step S80) is performed. The physical simulation (step S100) is a process of calculating the characteristics of a porous body by a physical calculation based on the three-dimensional structure generated by the structure generation process in step S170. The AI calculation (step S80) is a process of predicting the characteristics of the porous body using a learning model of a three-dimensional structure. In this manner, it is possible to appropriately perform the physical simulation and the AI calculation and determine the three-dimensional structure of the porous body that will provide better characteristics.
(5) It is also possible to manufacture a porous body (steps S330 to S360) using three-dimensional shape data based on the three-dimensional structure of the porous body determined by the porous body design method described above. In this manner, it is possible to rapidly and easily create a prototype of the porous body designed by the porous body design apparatus 100.

(Second embodiment)

Next, a second embodiment of the present invention will be described. In the first embodiment, description has been given of an example in which an optimal connectivity of a PN model representing a three-dimensional structure of a porous body is searched for using a learning model in which structural descriptors that contribute highly to the characteristics of the porous body, among structural descriptors representing the three-dimensional structure of the porous body, are used as input parameters. For this purpose, the three-dimensional structure of the porous body is reconfigured from a PN model corresponding to a network gene vector after a gene evolution process, and input parameters calculated on the basis of the three-dimensional structure are input to a learning model to perform characteristic prediction for a porous body using an AI calculation. On the other hand, in this embodiment, description will be given below of an example in which the characteristics of a porous body are directly predicted by an AI calculation from a PN model representing a three-dimensional structure of the porous body.

A porous body design apparatus according to this embodiment has the same configuration as that shown in Fig. 1 described in the first embodiment. Thus, the porous body design apparatus according to this embodiment will be described below using the configuration of the porous body design apparatus 100 shown in Fig. 1.

Fig. 6 is a flowchart showing a flow of processing of the porous body design apparatus according to the second embodiment of the present invention. The porous body design apparatus 100 according to this embodiment searches for a three-dimensional structure of a porous body having desired characteristics by repeatedly executing the processing shown in the flowchart in Fig. 6 by a control unit 1 in response to a user's operation input. Thereby, the design of a porous body which is performed by the user is supported.

In the flowchart in Fig. 6, the parts performing the same processing as in the flowchart in Fig. 2 described in the first embodiment are given the same step numbers as in Fig. 2. Thus, hereinafter, processing contents with step numbers different from those in Fig. 2 will be mainly described, and the description of common processes will be omitted unless otherwise necessary.

In step S20A, the control unit 1 performs an initial learning model generation process for setting the initial state of the learning model data 23. Here, a large number of samples having a three-dimensional structure of a porous body are acquired using the learning model creation unit 11 and the physical simulation unit 13, and a relationship between input parameters and characteristics that can be directly calculated from PN models obtained from the sample is obtained. Then, the initial state of the learning model data 23 can be set by creating a learning model on the basis of the obtained relationship. Details of an initial learning model generation process in this embodiment will be described later with reference to Fig. 7.

In step S50A, the control unit 1 generates an initial value of a PN model to which a genetic algorithm is applied. Here, the initial value of the PN model is generated by, for example, adding or deleting throats, or changing the number and positions of pores in the PN model of the target porous body created in step S40. At this time, as in step S50 in Fig. 2, the PN model of the target porous body is randomly changed by a preset population number (the number of generation individuals) P and set as the initial value of the PN model. In the following description, the porous body represented by the initial value of the PN model is referred to as a porous body of a 0-th generation.

In this embodiment, the initial value of the PN model is set in step S50A, and the information is stored in the storage unit 2 as PN model data 22. Then, the processing proceeds to step S60A without performing the process of step S55 in Fig. 2.

In step S60A, the control unit 1 calculates the values of input parameters that can be calculated from the PN model of each porous body of a j-th generation obtained in step S50A (when j = 0) or in step S160A (when j ≥ 1) to be described later. Here, among the structural descriptors described above, such as a pore-throat ratio and a pore-throat coordination number, which are related to the PN model, each structural descriptor that is selected in advance by the learning model creation unit 11 as a structural descriptors highly contributing to the characteristics of the porous body is used as an input parameter of the learning model, thereby calculating combinations of input parameter values that can be calculated from the PN model of the porous body of the j-th generation obtained in step S50A or step S160A. When the input parameter values calculated from the PN model of the porous body of the j-th generation are stored in the storage unit 2 as input parameter data 24, the processing proceeds to step S70. Then, in step S70, it is determined whether the current value of a variable i is a predetermined value M, and the processing proceeds to step S80A when i < M and proceeds to step S95 when i = M.

In step S80A, the control unit 1 causes the AI calculation unit 14 to perform characteristic prediction of an AI calculation using the learning model on the basis of the input parameter values calculated in step S60A. Here, the learning model data 23 is read out to obtain the learning model, and the input parameter values calculated from the PN model of the porous body of the j-th generation are input to the learning model to perform an AI calculation related to characteristic prediction of the porous body. Thereby, characteristic prediction for the porous body of the j-th generation is performed by obtaining predicted characteristic values corresponding to the input parameters calculated from the PN model. When the predicted characteristic values obtained for the porous body of the j-th generation are stored in the storage unit 2 as characteristic data 25, the processing proceeds to step S90.

In step S95, the control unit 1 causes the PN model conversion unit 12 to reconfigure the three-dimensional structure of the porous body of the j-th generation from the PN model. Here, the PN model data 22 representing the PN model of the porous body of the j-th generation generated by the gene evolution process performed in step S160A in the previous processing is read out from the storage unit 2, and the position and size of each pore in the PN model and the throats connecting the pores are reproduced on the porous body design apparatus 100. This processing can be realized using a well-known algorithm such as pix2pix, similar to step S55 and step S170 in Fig. 2 described in the first embodiment. Thereby, the PN model after the gene evolution process is inversely converted into a three-dimensional structure of each porous body of the j-th generation, and a three-dimensional structure of a porous body of the j-th generation having a different connectivity between pores from the three-dimensional structure of the target porous body can be virtually generated on the porous body design apparatus 100. When information on the three-dimensional structure of each porous body of the j-th generation reconfigured in this manner is recorded in the porous body structure data 21, the processing proceeds to step S100.

When it is determined in step S140 that j < N, in step S160A, the control unit 1 causes the gene evolution unit 16 to generate the next candidate PN model. Here, a gene evolution process is performed on the basis of the PN model of the porous bodies selected as parent porous bodies of the next generation in the immediately preceding step S135, and a PN model of child porous bodies for the parent porous bodies is created, thereby generating the next candidate PN model. Specifically, for example, by using a well-known calculation method such as a genetic algorithm, the PN models of the porous bodies of the j-th generation selected in step S135 are multiplied together to generate the PN model of a new porous body corresponding to their child as the (j + 1)-th generation PN model. By repeatedly performing such an operation for the combinations of the plurality of parent porous bodies, any number of PN models of child porous bodies of the next generation are generated. For example, P pairs of parent porous bodies of the j-th generation (P is a population number) are set, and P PN models of child porous bodies of the (j + 1)-th generation are created from these pairs as the next candidate PN models. As a result, it is possible to obtain, by the population number P, three-dimensional structures of porous bodies of the (j + 1)-th generation in which connectivity between pores is different with respect to the three-dimensional structure of the porous body of the j-th generation.

In this embodiment, when the process of step S160A is performed and the PN model after the gene evolution process is recorded in the PN model data 22, the processing proceeds to step S180 without performing the process of step S170 in Fig. 2. Then, when 1 is added to the value of the variable j in step S180, the processing returns to step S60A, and the process of step S60A and the subsequent processes are repeated again. Thereby, characteristic values are predicted or calculated by an AI calculation or a physical simulation on the basis of the PN model after the gene evolution process. Then, by adding a plot of the obtained characteristic prediction/calculation results on the graph, new evaluation results for characteristic values of porous bodies of the next generation are acquired in addition to the evaluation results for the characteristic values of the porous bodies of the previous generation which have been obtained up to the previous process, and parent porous bodies of the next generation to be used in the gene evolution process are selected from the obtained evaluation results. This series of processes realizes an optimization process for changing connectivity between pores in a three-dimensional structure of a porous body to search for an optimal connectivity between pores.

Fig. 7 is a flowchart showing details of the initial learning model generation process according to the second embodiment of the present invention, which is executed in step S20A in Fig. 6.

In the flowchart of Fig. 7, the parts performing the same processing as in the flowchart in Fig. 3 described in the first embodiment are given the same step numbers as in Fig. 3. Thus, hereinafter, processing contents with step numbers different from those in Fig. 3 will be mainly described, and the description of common processes will be omitted unless otherwise necessary.

In step S210A, the control unit 1 causes the PN model conversion unit 12 to convert the three-dimensional structure determined in step S210 into a PN model. Here, a three-dimensional structure of a porous body previously set as a sample can be converted into a PN model by the same method as in step S40 of Fig. 6.

In step S220A, the control unit 1 causes the learning model creation unit 11 to calculate the values of the structural descriptors corresponding to the PN model obtained by converting the three-dimensional structure in step S210. Here, as described above, for example, regarding various structural descriptors related to a PN model such as a pore-throat ratio and a pore-throat coordination number, the values of the structural descriptors for the PN model obtained in step S210 are calculated.

In step S260A, the control unit 1 causes the learning model creation unit 11 to create an initial learning model on the basis of the input parameters of the PN model selected in step S250 among the structural descriptors calculated in step S220A and the predicted characteristic values of the porous body obtained in step S230. Here, an initial learning model can be created by performing a model learning process using, for example, a well-known method on the basis of a relationship between the input parameters corresponding to the PN model of each sample and the predicted characteristic values.

When the initial learning model has been created in step S260A, the control unit 1 records the contents of the initial learning model in the learning model data 23 and ends the initial learning model generation process shown in the flowchart of Fig. 7. Thereafter, the processing proceeds to step S30 of Fig. 6.

According to the second embodiment of the present invention described above, in the characteristic prediction process of step S80A, the characteristics of the porous body are predicted on the basis of the initial value of the PN model set in step S50A, or the PN model in which throats have been added or deleted in the previous processing by the process of step S160A. In this manner, it is possible to predict the characteristics of a porous body in which connectivity between pores has been changed by an optimization process with less processing time.

(Third embodiment)

Next, a third embodiment of the present invention will be described. In this embodiment, description will be given of an example in which characteristic prediction for a porous body is performed by a calculation using a mathematical expression from a PN model that represents a three-dimensional structure of a porous body.

When considering a flow rate between any two pores in the PN model, if it is a saturated flow and a fluid viscosity $\mu$ is constant, a conductance G can be expressed by the following Formula (1) using a flow resistance R and the fluid viscosity $\mu$.

$$G = 1 / (R\mu) \ldots (1)$$

From the above Formula (1), when a pressure vector of one pore is $P_i$ and a pressure vector of the other pore is $P_j$, a flow rate between these two pores can be expressed by the following Formula (2).

$$Q_{ij} = G_{ij}(P_i - P_j) \quad \ldots \quad (2)$$

Further, in a three-dimensional structure of a porous body represented by a PN model, a pressure in pores can be obtained by solving an equation expressed by the following Formula (3).

$$GP = b \quad \ldots \quad (3)$$

In Formula (3), G is a modified diagonal element of an adjacency matrix weighted by a conductance, and is obtained by replacing the diagonal elements of boundary pores with 1, and the other elements with the sum of the rows multiplied by -1. In this manner, a pressure boundary condition and a mass conservation equation can be incorporated. In addition, P is a pore pressure vector, and b is a vector in which rows of boundary pores have a value of designated pressure, and the rest have a value of 0. The pore pressure can be obtained by solving Formula (3), and a permeability, which is one of the characteristics of a porous body, can be calculated from the PN model by calculating a flow rate using Formula (2).

Although an example in which a permeability is obtained by a calculation has been described above, it is possible to calculate other characteristics from a PN model by using a calculation formula corresponding to each characteristic. By combining this with the optimization process of a PN model described in the second embodiment, it is possible to determine a three-dimensional structure of a porous body that provides good characteristics.

According to the third embodiment of the present invention described above, by performing calculations using a predetermined calculation formula, it is possible to provide a useful technology that makes it possible to determine an optimal structure early in the design of a porous body without performing an AI calculation using a learning model.

The present invention is not limited to the above-described embodiment, and can be implemented using any components within the scope of the gist of the invention.

The above-described embodiments and modification examples are merely examples, and the present invention is not limited to these contents as long as the characteristics of the invention are not impaired. Furthermore, although various embodiments and modification examples have been described above, the present invention is not limited to these contents. Other aspects conceivable within the scope of the technical idea of the present invention are also included in the scope of the present invention.

[Reference Signs List]

| | |
|---|---|
| 1 | Control unit |
| 2 | Storage unit |
| 3 | Memory |
| 4 | Operation input apparatus |
| 5 | Display apparatus |
| 6 | Bus |
| 11 | Learning model creation unit |
| 12 | PN model conversion unit |
| 13 | Physical simulation unit |
| 14 | AI calculation unit |
| 15 | Characteristic evaluation unit |
| 16 | Gene evolution unit |
| 17 | Learning model update unit |
| 18 | Data conversion unit |
| 21 | Porous body structure data |
| 22 | PN model data |
| 23 | Learning model data |
| 24 | Input parameter data |
| 25 | Characteristic data |
| 26 | Characteristic evaluation data |
| 27 | Three-dimensional shape data |
| 100 | Porous body design apparatus |

**Claims**

1. A porous body design method, which is a design method of porous body using a computer, causing the computer to execute, a plurality of times:

   a characteristic prediction/calculation process of predicting or calculating characteristics of the porous body having a predetermined three-dimensional structure;
   an evaluation process of evaluating the characteristics of the porous body predicted or calculated by the characteristic prediction/calculation process; and
   an optimization process of changing connectivity between pores in the three-dimensional structure to search for an optimal connectivity, wherein the three-dimensional structure of the porous body is determined on the basis of evaluation results of the characteristics of the porous body by the evaluation process.

2. The porous body design method according to claim 1, causing the computer to execute a PN modeling process of generating a PN model expressing the three-dimensional structure by using a plurality of pores and a plurality of throats coupling the pores, wherein
   in the optimization process, the connectivity is changed by adding or deleting the throats in the PN model generated by the PN modeling process.

3. The porous body design method according to claim 2, wherein
   in the characteristic prediction/calculation process, the characteristics of the porous body are predicted or calculated on the basis of the PN model.

4. The porous body design method according to claim 2 or 3, causing the computer to execute a structure generation process of virtually generating, on the computer, the three-dimensional structure of the porous body based on the PN model to which the throats have been added or deleted in the optimization process, wherein
in the characteristic prediction/calculation process, the characteristics of the porous body having the three-dimensional structure generated in the structure generation process are predicted or calculated.

5. The porous body design method according to claim 4, wherein
in the characteristic prediction/calculation process, at least one of a physical simulation for calculating the characteristics of the porous body by a physical calculation based on the three-dimensional structure generated by the structure generation process, and an AI calculation for predicting the characteristics of the porous body using a learning model of the three-dimensional structure, is performed.

6. A porous body manufacturing method of manufacturing the porous body using three-dimensional shape data based on the three-dimensional structure of the porous body determined by the porous body design method according to any one of claims 1 to 3.

7. A porous body manufacturing method of manufacturing the porous body using three-dimensional shape data based on the three-dimensional structure of the porous body determined by the porous body design method according to claim 4.

8. A porous body manufacturing method of manufacturing the porous body using three-dimensional shape data based on the three-dimensional structure of the porous body determined by the porous body design method according to claim 5.

# FIG. 1

PORITUM BODY DESIGN APPARATUS 100

| 6 |
| --- |

**CONTROL UNIT** 1

| LEARNING MODEL CREATION UNIT | 11 |
| PN MODEL CONVERSION UNIT | 12 |
| PHYSICAL SIMULATION UNIT | 13 |
| AI CALCULATION UNIT | 14 |
| CHARACTERISTIC EVALUATION UNIT | 15 |
| GENE EVOLUTION UNIT | 16 |
| LEARNING MODEL UPDATE UNIT | 17 |
| DATA CONVERSION UNIT | 18 |

**STORAGE UNIT** 2

| POROUS BODY STRUCTURE DATA | 21 |
| PN MODEL DATA | 22 |
| LEARNING MODEL DATA | 23 |
| INPUT PARAMETER DATA | 24 |
| CHARACTERISTIC DATA | 25 |
| CHARACTERISTIC EVALUATION DATA | 26 |
| THREE-DIMENSIONAL SHAPE DATA | 27 |

MEMORY 3

OPERATION INPUT APPARATUS 4

DISPLAY APPARATUS 5

FIG. 2

```
                    ┌──────────┐
                    │  START   │
                    └────┬─────┘
                         ▼
         ┌───────────────────────────┐  S10
         │        i=0, j=0           │
         └───────────┬───────────────┘
                     ▼
         ┌───────────────────────────┐  S20
         │  INITIAL LEARNING MODEL   │
         │    GENERATION PROCESS     │
         └───────────┬───────────────┘
                     ▼
         ┌───────────────────────────┐  S30
         │  ACQUIRE THREE-DIMENSIONAL│
         │    STRUCTURE OF TARGET    │
         │       POROUS BODY         │
         └───────────┬───────────────┘
                     ▼
         ┌───────────────────────────┐  S40
         │  CONVERT THREE-DIMENSIONAL│
         │   STRUCTURE INTO PN MODEL │
         └───────────┬───────────────┘
                     ▼
         ┌───────────────────────────┐  S50
         │    SET INITIAL VALUES OF  │
         │   NETWORK GENE VECTOR     │
         └───────────┬───────────────┘
                     ▼
         ┌───────────────────────────┐  S55
         │    RECONFIGURE THREE-     │
         │   DIMENSIONAL STRUCTURE   │
         └───────────┬───────────────┘
                     ▼
         ┌───────────────────────────┐  S60
         │  CALCULATE VALUES OF INPUT│
         │        PARAMETERS         │
         └───────────┬───────────────┘
                     ▼
                  ◇ S70                    YES
              ◇  i=M?  ◇ ─────────────────────┐
                  ◇                           │
                  │ NO                        ▼
                  ▼                ┌─────────────────────────┐ S100
         ┌───────────────────────┐│ PERFORM CHARACTERISTIC  │
         │ PERFORM CHARACTERISTIC ││ CALCULATION BY PHYSICAL │
         │ PREDICTION BY AI       ││      SIMULATION         │
         │ CALCULATION       S80  │└───────────┬─────────────┘
         └───────────┬───────────┘            ▼
                     ▼                ┌─────────────────────────┐ S110
         ┌───────────────────────┐    │  UPDATE LEARNING MODEL  │
         │       i=i+1      S90   │    └───────────┬─────────────┘
         └───────────┬───────────┘                ▼
                     │              ┌─────────────────────────┐ S120
                     │              │          i=0            │
                     │              └───────────┬─────────────┘
                     ◄──────────────────────────┘
                     ▼
         ┌───────────────────────────┐ S130
         │  PLOT CHARACTERISTIC      │
         │  PREDICTION/CALCULATION   │
         │        RESULTS            │
         └───────────┬───────────────┘
                     ▼
         ┌───────────────────────────┐ S135
         │  SELECT PARENT POROUS     │
         │  BODIES OF NEXT GENERATION│
         └───────────┬───────────────┘
                     ▼
                  ◇ S140              NO
              ◇  j=N?  ◇ ──────────────────────┐
                  ◇                            ▼
                  │ YES           ┌─────────────────────────┐ S160
                  ▼               │    GENERATE NEXT        │
         ┌───────────────────────┐│ CANDIDATE NETWORK       │
         │ DETERMINE THREE-       ││    GENE VECTOR          │
         │ DIMENSIONAL STRUCTURE  │└───────────┬─────────────┘
         │ OF POROUS BODY    S150 │            ▼
         └───────────┬───────────┘ ┌─────────────────────────┐ S170
                     ▼              │    RECONFIGURE THREE-   │
              ┌──────────┐          │  DIMENSIONAL STRUCTURE  │
              │   END    │          └───────────┬─────────────┘
              └──────────┘                      ▼
                                     ┌─────────────────────────┐ S180
                                     │         j=j+1           │
                                     └─────────────────────────┘
```

# FIG. 3

```
┌─────────────────────────────┐
│  INITIAL LEARNING MODEL     │
│  GENERATION PROCESS         │
└─────────────────────────────┘
              │
              ▼
┌─────────────────────────────┐
│ DETERMINE THREE-DIMENSIONAL │ S210
│ STRUCTURE OF POROUS BODY    │
└─────────────────────────────┘
              │
              ▼
┌─────────────────────────────┐
│ CALCULATE STRUCTURAL        │ S220
│ DESCRIPTORS                 │
└─────────────────────────────┘
              │
              ▼
┌─────────────────────────────┐
│ PERFORM PHYSICAL SIMULATION │ S230
└─────────────────────────────┘
              │
              ▼
          ◇ S240
   HAS PREDETERMINED
   NUMBER OF SAMPLES        NO
   BEEN PROCESSED?
              │ YES
              ▼
┌─────────────────────────────┐
│ SELECT, AS INPUT PARAMETERS,│ S250
│ STRUCTURAL DESCRIPTORS      │
│ THAT ARE HIGHLY CORRELATED  │
│ WITH CHARACTERISTICS        │
└─────────────────────────────┘
              │
              ▼
┌─────────────────────────────┐
│ CREATE INITIAL LEARNING MODEL│ S260
└─────────────────────────────┘
              │
              ▼
         ( RETURN )
```

# FIG. 4

$$A = \begin{pmatrix} 1 \\ 0 \\ 1 \\ 0 \\ 0 \\ 1 \\ 0 \end{pmatrix}$$

# FIG. 5

START

CREATE THREE-DIMENSIONAL SHAPE DATA — S310

INPUT THREE-DIMENSIONAL SHAPE DATA TO 3D PRINTER — S320

START TO INJECT MATERIAL — S330

MOVE HEAD OF 3D PRINTER TO COORDINATE POSITION AND INJECT MATERIAL — S340

HAS MATERIAL BEEN INJECTED FOR ALL STRUCTURES OF THREE-DIMENSIONAL SHAPE DATA? — S350

NO

YES

END INJECTION OF MATERIAL — S360

END

FIG. 6

# FIG. 7

INITIAL LEARNING MODEL
GENERATION PROCESS

DETERMINE THREE-DIMENSIONAL
STRUCTURE OF POROUS BODY — S210

CONVERT THREE-DIMENSIONAL
STRUCTURE INTO PN MODEL — S210A

CALCULATE STRUCTURAL
DESCRIPTORS CORRESPONDING
TO PN MODEL — S220A

PERFORM PHYSICAL SIMULATION — S230

HAS PREDETERMINED
NUMBER OF SAMPLES BEEN
PROCESSED? — S240
NO

YES

SELECT, AS INPUT PARAMETERS,
STRUCTURAL DESCRIPTORS
THAT ARE HIGHLY CORRELATED
WITH CHARACTERISTICS — S250

CREATE INITIAL LEARNING MODEL — S260A

RETURN

<table>
<tr><td colspan="2">INTERNATIONAL SEARCH REPORT</td><td>International application No.<br><br>**PCT/JP2023/010484**</td></tr>
</table>

**A. CLASSIFICATION OF SUBJECT MATTER**

*G06F 30/27*(2020.01)i; *C04B 38/00*(2006.01)i; *G06F 30/10*(2020.01)i
FI: G06F30/27; G06F30/10 100; C04B38/00 303Z

According to International Patent Classification (IPC) or to both national classification and IPC

**B. FIELDS SEARCHED**

Minimum documentation searched (classification system followed by classification symbols)

G06F30/27; C04B38/00; G06F30/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2019-96225 A (TOYOTA MOTOR CORP.) 20 June 2019 (2019-06-20)<br>paragraphs [0017]-[0038] | 1-8 |
| A | JP 2010-128746 A (TOYOTA MOTOR CORP.) 10 June 2010 (2010-06-10)<br>paragraphs [0022]-[0051] | 1-8 |
| A | CN 112926265 A (ZHUHAI FUDAN INNOVATION RESEARCH INSTITUTE) 08 June 2021 (2021-06-08)<br>entire text, all drawings | 1-8 |
| A | CN 113139313 A (CHONGQING UNIVERSITY) 20 July 2021 (2021-07-20)<br>entire text, all drawings | 1-8 |
| A | US 2019/0005172 A1 (UNIVERSITY OF CINCINNATI) 03 January 2019 (2019-01-03)<br>entire text, all drawings | 1-8 |

☐ Further documents are listed in the continuation of Box C.  ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **26 April 2023** | **16 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/010484**

| Patent document cited in search report | | | Publication date (day/month/year) | Patent family member(s) | Publication date (day/month/year) |
|---|---|---|---|---|---|
| JP | 2019-96225 | A | 20 June 2019 | (Family: none) | |
| JP | 2010-128746 | A | 10 June 2010 | (Family: none) | |
| CN | 112926265 | A | 08 June 2021 | (Family: none) | |
| CN | 113139313 | A | 20 July 2021 | (Family: none) | |
| US | 2019/0005172 | A1 | 03 January 2019 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6940786 B

**Non-patent literature cited in the description**

- **TOMOKI YASUDA ; SHINICHI OOKAWARA ; SHIRO YOSHIKAWA ; HIDEYUKI MATSUMOT**. Machine learning and data-driven characterization framework for porous materials: Permeability prediction and channeling defect detection. *Chemical Engineering Journal*, 2021, vol. 420, 130069